# EUROPÄISCHE PATENTSCHRIFT

(11) **EP 1 654 266 B1**
(45) Veröffentlichungstag und Bekanntmachung des Hinweises auf die Patenterteilung: **04.06.2008**
(21) Anmeldenummer: 04741285.3
(22) Anmeldetag: 23.07.2004
(51) Int. Cl.: C07F 15/00, C07C 69/712, C08F 290/00

(54) **NEUE METATHESEKATALYSATOREN**
NOVEL METATHESIS CATALYSTS
NOUVEAUX CATALYSEURS DE METATHESE

(30) Priorität: 02.08.2003 DE 10335416
(43) Veröffentlichungstag der Anmeldung: 10.05.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: ARLT, Dieter, 32657 Lemgo (DE)
(74) Vertreter: Hammann, Heinz
(86) Internationale Anmeldenummer: PCT/EP2004/008388
(87) Internationale Veröffentlichungsnummer: WO 2005/016944

(56) Entgegenhaltungen:
- WO-A-02/14376
- AHMAD-JUNAN, S. ASIAH ET AL: "Aryloxymethyl radical cyclizations mimicking biological carbon-carbon bond formation to methoxy groups" JOURNAL OF THE CHEMICAL SOCIETY, PERKIN TRANSACTIONS 1: ORGANIC AND BIO-ORGANIC CHEMISTRY (1972-1999) , Bd. 18, 1992, Seiten 2313-2320, XP002302739 ISSN: 0300-922X
- DATABASE CA [Online] CHEMICAL ABSTRACTS SERVICE, COLUMBUS, OHIO, US; WYRZYKIEWICZ, ELZBIETA ET AL: "N-(E)-2-stilbenyloxymethylenecarbonyl substituted hydrazones of ortho-, meta-, and para-hydroxybenzaldehydes" retrieved from STN Database accession no. 134:131292 XP002302742 & BULLETIN OF THE POLISH ACADEMY OF SCIENCES, CHEMISTRY , Bd. 48, Nr. 3, 2000, Seiten 213-229, ISSN: 0239-7285
- TIETZE, LUTZ F. ET AL: "Intra- and intermolecular hetero-Diels-Alder reactions. 17. Intramolecular hetero-Diels-Alder reaction of alkylidene- and benzylidenepyrazolones and benzylideneisoxazolones. Investigations toward the conformation of the transition state" JOURNAL OF ORGANIC CHEMISTRY , Bd. 53, Nr. 4, 1988, Seiten 810-820, XP002302740 ISSN: 0022-3263
- DATABASE CA [Online] CHEMICAL ABSTRACTS SERVICE, COLUMBUS, OHIO, US; "Addition reactions of unsaturated compounds" retrieved from STN Database accession no. 102:8602 XP002302743 & JP 59 118721 A2 (NIPPON ZEON CO., LTD., JAPAN) 9. Juli 1984 (1984-07-09)
- DATABASE CA [Online] CHEMICAL ABSTRACTS SERVICE, COLUMBUS, OHIO, US; PANARIN, E. F. ET AL: "Derivatives of o-vinylphenol. Synthesis of .alpha.-(o-vinylphenoxy) carboxylic acids" retrieved from STN Database accession no. 85:20772 XP002302744 & DEPOSITED DOC. , VINITI 5598-73, 5 PP. AVAIL.: BLLD, 1973,
- DATABASE CAPLUS [Online] CHEMICAL ABSTRACTS SERVICE, COLUMBUS, OHIO, US; NEWSOROFF, G. P. ET AL: "Nuclear magnetic resonance spectra of .alpha.,.beta.,.beta.- trimethylstyrenes" retrieved from STN Database accession no. 65:90114 XP002302838 & AUSTRALIAN JOURNAL OF CHEMISTRY , Bd. 19, Nr. 9, 1966, Seiten 1667-1675, ISSN: 0004-9425

## Beschreibung

Die vorliegende Erfindung betrifft neue Verbindungen der Formel **1**, ihre Darstellung, und der Einsatz der Verbindungen der Formel **1** als Katalysatoren bei verschiedenen Metathesereaktionen

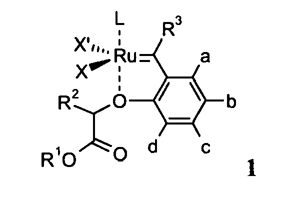

### HINTERGRUND DER ERFINDUNG

Rutheniumkomplexe der Formel **A** sind aus WO 02/14376 A2 bekannt und werden als aktive, luftstabile und zurückgewinnbaren Metathesekatalysatoren beschreiben. Es sind weiterhin Katalysatoren dieser Art bekannt geworden, (Angew. Chem. 2002, 114, Nr.5, 832-834; Angew. Chem. 2002, 114, Nr.13, 2509-2511; Angew. Chem. 2002, 114, Nr. 21, 4210-4212), die durch die Formeln **B, C** und **D** beschrieben werden, die eine noch höhere Aktivität als A besitzen.

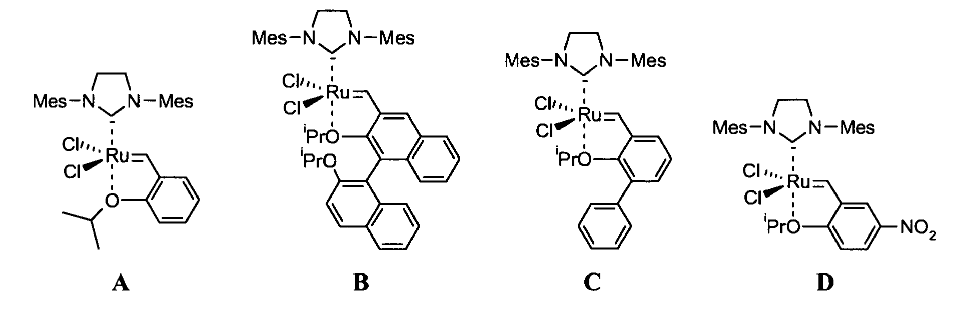

Die Verbesserung der Aktivität von **B, C** und **D** im Vergleich mit **A** wird auf sterische und elektronische Effekte der Substituenten am Phenylkern des Isopropoxy-phenylmethylen-Liganden zurückgeführt. Es wurde nun überraschenderweise gefunden, dass eine hohe Aktivitätssteigerung von Metathesekatalysatoren des Typs **A** durch eine spezielle Veränderung des aliphatischen Anteiles der Ethergruppe zu erreichen ist.

### DETAILLIERTE BESCHREIBUNG DER ERFINDUNG

Gegenstand der vorliegenden Erfindung sind neue Ruthenium-Komplexe der Formel 1 und ihre Verwendung als Metathesekatalysatoren, 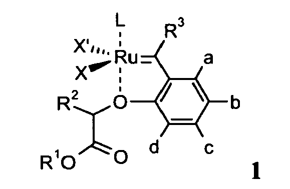 worin
- X und X': anionische Liganden; bevorzugt Halogen, besonders bevorzugt Cl oder Br;
- L: neutraler Ligand;
- a, b, c, d: unabhängig voneinander H, -NO₂, C₁₋₁₂-Alkyl, C₁₋₁₂-Alkoxy oder Phenyl, wobei Phenyl gegebenenfalls mit einem Rest ausgewählt aus der Gruppe C₁₋₆-Alkyl und C₁₋₆-Alkoxy substituiert sein kann;
- R¹: C₁₋₁₂-Alkyl, C₅₋₆-Cycloalkyl, C₇₋₁₈-Aralkyl, Aryl;
- R²: H, C₁₋₁₂-Alkyl, C₅₋₆-Cycloalkyl, C₇₋₁₈-Aralkyl, Aryl;
- R³: H, C₁₋₁₂-Alkyl, C₂₋₁₂-Alkenyl, C₂₋₁₂-Alkinyl, Aryl;
bedeuten. Bevorzugt sind hierbei die Verbindungen der Formel **1**, worin
- X und X': Halogen;
- L: neutraler Ligand;
- a, b, c, d: unabhängig voneinander H, -NO₂, C₁₋₆-Alkyl, C₁₋₆-Alkoxy oder Phenyl, wobei Phenyl gegebenenfalls mit einem Rest ausgewählt aus der Gruppe C₁₋₄-Alkyl und C₁₋₄-Alkoxy substituiert sein kann;
- R¹: C₁₋₆-Alkyl, C₅₋₆-Cycloalkyl, C₇₋₁₁-Aralkyl, Aryl;
- R²: H, C₁₋₆-Alkyl, C₅₋₆-Cycloalkyl, C₇₋₁₁-Aralkyl, Aryl;
- R³: H, C₁₋₆-Alkyl, C₂₋₆-Alkenyl, C₂₋₆-Alkinyl, Aryl;
bedeuten, wovon besonders bevorzugt die Verbindungen der Formel 1 sind, worin
- X und X': Cl oder Br;
- L: neutraler Ligand;
- a, b, c, d: unabhängig voneinander H, -NO₂, Methyl, Ethyl, *iso*-Propyl, Methoxy, oder Phenyl, wobei Phenyl gegebenenfalls mit einem Rest ausgewählt aus der Gruppe Methyl und Methoxy substituiert sein kann;
- R¹: Methyl, Ethyl , n-Propyl, iso-Propyl, n-Butyl, sec-Butyl, tert-Butyl, n-Heptyl, Cyclopentyl, Cyclohexyl, 2-Methylcyclohexyl, 2,4-Dimethylcyclohexyl Benzyl, 1-Phenylethyl, 2-Phenylethyl, Phenyl, o-, m-, p-Tolyl und 3,5-Dimethylphenyl.
- R²: H, Methyl, Ethyl , n-Propyl, iso-Propyl, n-Butyl, sec-Butyl, tert-Butyl, n-Heptyl, Cyclopentyl, Cyclohexyl, 2-Methylcyclohexyl, 2,4-Dimethylcyclohexyl Benzyl, 1-Phenylethyl, 2-Phenylethyl, Phenyl, o-, m-, p-Tolyl und 3,5-Dimethylphenyl.
- R³: H, Methyl, Ethyl, Phenyl
bedeuten. Von den oben genannten Verbindungen der allgemeinen Formel 1 sind diejenigen besonders bevorzugt, in denen R¹, R², R³, X, X' und L die genannten Bedeutungen haben können und
- a, b, c: H; und
- d: Phenyl mit einem Rest ausgewählt aus der Gruppe C₁₋₆-Alkyl und C₁₋₆-Alkoxy substituiert sein kann;
oder
- a, c, d: H; und
- b: -NO₂;
bedeuten. Weiterhin sind von den oben genannten Verbindungen der allgemeinen Formel 1 diejenigen besonders bevorzugt, in denen R¹, R², R³, X, X', a, b, c und d die genannten Bedeutungen haben können und
- L: P(R⁴)₃ oder ein Liganden der Formel L¹, L², L³ oder L⁴ bedeutet, worin 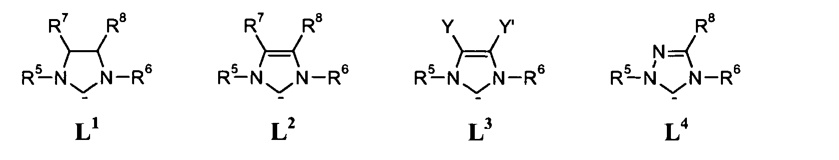
- R⁴: C₁₋₆-Alkyl, Cycloalkyl oder Aryl
- R⁵ und R⁶: unabhängig voneinander H, C₁₋₆-Alkyl oder Aryl;
- R⁷ und R⁸: unabhängig voneinander H, C₁₋₆-Alkyl, C₂₋₆-Alkenyl oder Aryl; oder
- R⁷ und R⁸: zusammen eine 3- oder 4-gliedrige Alkylenbrücke bilden; und
- Y und Y': Halogen; bevorzugt Cl oder Br bedeutet.

Am meisten bevorzugt sind Verbindungen der allgemeinen Formel 1, worin
- X und X': Cl;
- L: L¹;
- a, b, c, d: H;
- R¹: Methyl;
- R²: Methyl;
- R³: H;
- R⁵ und R⁶: Mesityl
- R⁷ und R⁸: H;
bedeutet. Die neuen Verbindungen der Formel 1 werden durch Umsetzung von Präliganden der Formel **2** mit Ruthenium-Komplexen der Formel **3** erhalten. 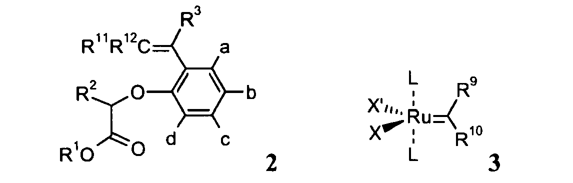 wobei R³, a, b, c und d die für Formel 1 angegebene Bedeutung haben und
- R¹: C₁₋₁₂-Alkyl, C₅₋₆-Cycloalkyl, C₇₋₁₈-Aralkyl, Aryl; bevorzugt C₁₋₆-Alkyl, C₅₋₆-Cycloalkyl, C₇₋₁₁-Aralkyl, Aryl;
- R²: H, C₁₋₁₂-Alkyl, C₅₋₆-Cycloalkyl, C₇₋₁₈-Aralkyl, Aryl; bevorzugt C₁₋₆-Alkyl, C₅₋₆-Cycloalkyl, C₇₋₁₁-Aralkyl, Aryl;
- R¹¹ und R¹²: unabhängig voneinander H, C₁₋₆-Alkyl, gegebenenfalls mit einem oder mehreren Halogen substituiert, oder Aryl, gegebenenfalls mit einem oder mehreren Halogen oder C₁₋₆-Alkyl substituiert; bevorzugt H, C₁₋₆-Alkyl oder Aryl; besonders und
- L: neutraler Ligand; bevorzugt L¹, L², L³ oder L⁴;
- R⁹ und R¹⁰: unabhängig voneinander H, C₁₋₆-Alkyl, gegebenenfalls mit einem oder mehreren Halogen substituiert, oder Aryl, gegebenenfalls mit einem oder mehreren Halogen oder C₁₋₆-Alkyl substituiert; bevorzugt H, C₁₋₆-Alkyl oder Aryl; und
bedeuten, mit der Maßgabe, dass R¹ und R² nicht gleichzeitig Methyl bedeuten können.

Die gezeigten Komplexe können als reine Enantionmere oder Enantiomerenpaare auftreten. Im Rahmen der Erfindung sind deshalb zusätzlich zu etwaigen Racematen ebenfalls die reine Enantiomere enthalten, die durch das Stereozentrum die Enantiomerie während der Katalyse auf ein Substrat übertragen können.

Ein zusätzlicher Aspekt der Erfindung ist ein Verfahren zur Durchführung von Metathesereaktionen, in dem zwei Verbindungen zur Reaktion gebracht werden, die je eine olefinische Doppelbindung enthalten oder eine der Verbindungen mindestens zwei olefinische Doppelbindungen enthält und in dem als Katalysator eine der oben genannten Verbindungen der Formel **1** verwendet wird, bzw. ein Verfahren zur Durchführung einer Ringschlussmetathese (RCM) oder einer Kreuzmetathese (CM) an dem eine Verbindung, die zwei olefinische Doppelbindungen enthält, als Substrat und eine der Verbindungen der Formel **1** als Katalysator beteiligt ist.

### VERWENDETE BEGRIFFE UND DEFINTIONEN

Unter einem "anionischen Liganden" (X oder X') werden im Rahmen der Erfindung negativ geladene Moleküle oder Atome mit Elektronendonor-Eigenschaften verstanden. Beispielhaft hierfür seinen hier Halogene, wie Fluor Chlor, Brom oder Iod genannt.

Unter einem "neutralen Liganden" (L) werden im Rahmen der Erfindung ungeladene oder ladungsneutral erscheinende Moleküle oder Atome mit Elektronendonor-Eigenschaften verstanden. Beispielhaft seinen hier tertiäre Phosphine, die aliphatische, cycloaliphatische und aromatische Kohlenwasserstoffreste enthalten wie Trioctylphosphin, Tridodecylphosphin, Tricyclohexylphosphin. Tris-(2-methylcyclohexyl)phosphin und Tris-(o-tolyl) phosphin genannt. Als besonders bevorzugte neutrale Liganden seien NHC-Liganden genannt wie z.B. die durch die Formeln L¹, L², L³ und L⁴ beschriebenen Verbindungen: 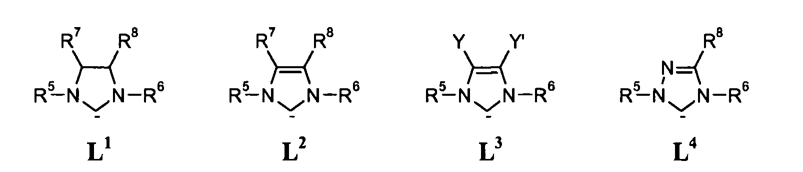 worin
- R⁵ und R⁶: unabhängig voneinander für H, C₁₋₆-Alkyl oder Aryl stehen,
- R⁷ und R⁸: unabhängig voneinander für H, C₁₋₆-Alkyl, C₁₋₆-Alkenyl oder Aryl stehen oder zusammen eine 3- oder 4-gliedrige Alkylenbrücke bilden und
- Y und Y': für Halogen stehen.

Unter dem Begriff "C₁₋₁₂-Alkyl" (auch soweit sie Bestandteil anderer Reste sind) werden verzweigte und unverzweigte Alkylgruppen mit 1 bis 12 Kohlenstoffatomen verstanden, dem entsprechend werden unter dem Begriff "C₁₋₆-Alkyl" verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 6 Kohlenstoffatomen, besonders bevorzugt 1 bis 4 Kohlenstoffatome. Beispielsweise werden hierfür genannt: Methyl, Ethyl, *n*-Propyl, *iso-*Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso-*Propyl, Butyl umfasst *iso*-Butyl, *sec*-Butyl und *tert*-Butyl etc.

Unter dem Begriff "C₂₋₁₂-Alkenyl" (auch soweit sie Bestandteil anderer Reste sind) werden verzweigte und unverzweigte Alkenylgruppen mit 2 bis 12 Kohlenstoffatomen verstanden, soweit sie mindestens eine Doppelbindung aufweisen. Dem entsprechend werden unter dem Begriff "C₂₋₆-Alkenyl" Alkenylgruppen mit 2 bis 6 Kohlenstoffatomen verstanden und unter dem Begriff "C₂₋₄-Alkenyl" verzweigte und unverzweigte Alkenylgruppen mit 2 bis 4 Kohlenstoffatomen verstanden. Bevorzugt sind Alkenylgruppen mit 2 bis 6 Kohlenstoffatomen, besonders bevorzugt 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₁₂-Alkinyl" (auch soweit sie Bestandteil anderer Reste sind) werden verzweigte und unverzweigte Alkinylgruppen mit 2 bis 12 Kohlenstoffatomen verstanden, soweit sie mindestens eine Dreifachbindung aufweisen. Dem entsprechend werden unter dem Begriff "C₂₋₆-Alkinyl" Alkinylgruppen mit 2 bis 6 Kohlenstoffatomen verstanden und unter dem Begriff "C₂₋₄-Alkinyl" verzweigte und unverzweigte Alkinylgruppen mit 2 bis 4 Kohlenstoffatomen verstanden. Bevorzugt sind Alkinylgruppen mit 2 bis 6 Kohlenstoffatomen, besonders bevorzugt 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₁₋₁₂-Alkoxy" (auch soweit sie Bestandteil anderer Reste sind) werden verzweigte und unverzweigte Alkoxygruppen mit 1 bis 12 Kohlenstoffatomen verstanden, dem entsprechend werden unter dem Begriff "C₁₋₆-Alkoxy" verzweigte und unverzweigte Alkoxygruppen mit 1 bis 6 Kohlenstoffatomen verstanden und unter dem Begriff "C₁₋₄-Alkoxy" verzweigte und unverzweigte Alkoxygruppen mit 1 bis 4 Kohlenstoffatomen verstanden. Bevorzugt sind Alkoxygruppen mit 1 bis 6 Kohlenstoffatomen, besonders bevorzugt 1 bis 4 Kohlenstoffatome. Beispielsweise werden hierfür genannt: Methoxy, Ethoxy, Propoxy, Butoxy oder Pentoxy. Gegebenenfalls werden für vorstehend genannten Gruppen auch die Abkürzungen MeO, EtO, PrO, etc. verwendet. Sofern nicht anders beschrieben, umfassen die Definitionen Propoxy, Butoxy und Pentoxy alle denkbaren isomeren Formen der jeweiligen Reste. So umfasst beispielsweise Propoxy *n*-Propoxy und *iso*-Propoxy, Butoxy umfasst *iso*-Butoxy, *sec*-Butoxy und *tert*-Butoxy etc.

Unter dem Begriff "C₅₋₆-Cycloalkyl" (auch soweit sie Bestandteil anderer Reste sind) werden cyclische Alkylgruppen mit 5 oder 6 Kohlenstoffatomen verstanden. Beispielsweise werden hierfür genannt: Cyclopentyl oder Cyclohexyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert-*Butyl, Hydroxy, Fluor, Chlor, Brom und Jod.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl. Soweit nicht anders beschreiben, können die Aromaten substituiert sein mit einem oder mehreren Resten ausgewählt aus der Gruppe bestehend aus Methyl, Ethyl, *iso*-Propyl, *tert-*Butyl*,* Hydroxy, Fluor, Chlor, Brom und Jod.

Unter dem Begriff C₇₋₁₈-Aralkyl" (auch soweit sie Bestandteil anderer Reste sind) werden verzweigte und unverzweigte Alkylgruppen mit 1 bis 8 Kohlenstoffatomen verstanden, die mit einem aromatische Ringsysteme mit 6 oder 10 Kohlenstoffatomen substituiert sind, entsprechend werden unter dem Begriff "C₇₋₁₁-Aralkyl" verzweigte und unverzweigte Alkylgruppen mit 1 bis 4 Kohlenstoffatomen verstanden, die mit einem aromatische Ringsysteme mit 6 Kohlenstoffatomen substituiert sind. Beispielsweise werden hierfür genannt: Benzyl, 1- oder 2-Phenylethyl. Soweit nicht anders beschreiben, können die Aromaten substituiert sein mit einem oder mehreren Resten ausgewählt aus der Gruppe bestehend aus Methyl, Ethyl, *iso*-Propyl, *tert-*B*u*tyl*,* Hydroxy, Fluor, Chlor, Brom und Jod.

### DARSTELLUNG DER VERBINDUNGEN

Die Umsetzung der Ruthenium-Komplexe der Formel 3 mit den Präliganden der Formel **2** wird in inerten Lösungsmitteln, z.B. CH₂Cl₂ bei ca. 0°bis 80° C ausgeführt. Vorteilhaft ist der Zusatz von CuCl zum Reaktionsgemisch. Die Reaktionspartner werden i. **a.** in äquivalenten Mengen eingesetzt, zur Erhöhung der Ausbeute kann aber auch die jeweils wertvollere Komponente im Unterschuss eingesetzt werden. Es kann auch zweckmäßig sein, den Komplex der Formel **3** aus anderen Rutheniumverbindungen und Ligandvorprodukten, z.B. Dihydroimidazoliniumsalzen, in situ zu erzeugen, um über die daraus entstehenden Komplexe der Formel **3** zu den neuen Metathesekatalysatoren der Formel **1** mit der jeweils gewünschten Ligandenkombination zu gelangen.

Die durch Ligandenaustauschreaktion hergestellten Metathesekatalysatoren der Formel **1** können von anderen Reaktionsprodukten, die in dem Reaktionsgemisch unlöslich sind, durch Filtration ihrer Lösung abgetrennt und nach Einengen der Lösung durch Chromatographie oder Kristallisation in reiner Form gewonnen werden. Es ist aber auch möglich, die Rohprodukte oder die in situ erzeugten Katalysatoren direkt zur Ausführung von Metathesereaktionen einzusetzen.

Die Präliganden der Formel **2** können in an sich bekannter Weise aus aromatischen Aldehyden, die entsprechende Substituenten enthalten, durch Olefinierungsreaktionen, z. B. durch Umsetzung mit Phosphor-Yliden nach Wittig hergestellt werden.
Besonders bevorzugt ist ein neues Kombinationsverfahren, das von ggf. entsprechend substituierten Phenylallylethern ausgeht, die durch Claisen-Umlagerung und katalytische Doppelbindungsisomerisierung zu ggf. entsprechend substituierten 2-Alkenyl-phenolen führt, die nachfolgend durch Alkylierung mit α-Halogencarbonsäureestern zu Verbindungen der Formel **2** umgesetzt werden.

Die Alkylierung von Phenolen zu Alkyl-phenylethern ist dem Fachmann wohlbekannt, sie wird üblicherweise in Lösungsmittel in Gegenwart basischer Stoffe durch Umsetzung mit nucleophilen Reagenzien ausgeführt. Die Umsetzung mit α-Halogencarbonsäureestern verläuft besonders glatt und mit guten Ausbeuten. Als Lösungsmittel kommen z. B. Alkohole wie Ethanol oder aprotische polare Lösungsmittel wie Dimethylformamid in Frage. Die Alkylierung kann auch unter Phasentransferbedingungen ausgeführt werden. Als basische Stoffe seien Alkalicarbonate genannt, ebenso können die Alkalisalze der Zwischenprodukte, die eine freie aromatisch gebundene OH-Gruppe enthalten, für diese Umsetzung verwendet werden.

Zur Erläuterung der Synthesesequenz, die bevorzugt für die Herstellung von Verbindungen der Formel **2** angewendet wird, sei als konkretes Beispiel die Herstellung von (3-Propenylbiphen-2-yl) (1-methoxycarbonyl-ethyl)ether beschrieben:

Ausgehend von 2-Hydroxybiphenyl wird durch Alkylierung mit Allylchlorid in DMF als Lösungsmittel in Gegenwart von Kaliumcarbonat 2-Allyloxy-biphenyl erhalten. Durch thermische Umlagerung bei 190° C in Trichlorbenzol wird anschließend 3-Allyl-2-hydroxy-biphenyl gewonnen, das katalytisch (RhCl₃ H₂O) in ethanolischer Lösung in Gegenwart von p-Toluolsulfonsäure zu einem E/Z-Gemisch von 3-Propenyl-2-hydroxybiphenyl umgelagert wird. Alkylierung dieses Zwischenproduktes mit 2-Brompropionsäuremethylester ergibt den Präliganden (3-Propenyl-biphen-2-yl)(1-methoxycarbonyl-ethyl)ether.

Die Verbindungen der Formel **1** sind hochaktive Metathesekatalysatoren, die mit Erfolg auch für die Durchführung schwierig verlaufender Metathesereaktionen eingesetzt werden können, wobei alle Arten dieser Reaktionen, (RCM, CM, ROMP etc.), einbezogen sind.

### BEISPIEL 1

### Herstellung eines Metathese-Katalysators der Formel 1a.

### 1.1) Herstellung des neuen Präliganden der Formel 2a:

Eine Mischung von 500 mg (3,72 mmol) 2-Propenylphenol (E/Z-Gemisch), hergestellt durch Claisen-Umlagerung von Phenylallylether und nachfolgende Doppelbindungsisomerisierung, 1,02 g Kaliumcarbonat (0,74 mmol) und 745 mg rac. 2-Brompropionsäuremethylester (4,46 mmol) und 10 ml Dimethylformamid wurde über Nacht bei RT und anschließend 4 Stunden bei 80° C gerührt. Die Reaktionsmischung wurde anschließend zu 40 ml Wasser gegeben und dreimal mit je 30 ml Diethylether extrahiert. Die organische Phase wurde mit 5%iger Natronlauge gewaschen, abgetrennt, mit Na₂SO₄ getrocknet u. eingedampft. Man erhielt 685 mg (83,6% d.Th.) nahezu reinen 2-(2-Propenyl-phenyloxy)propionsäuremethylester.

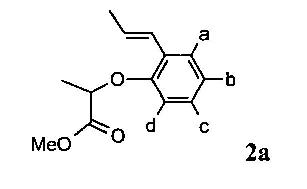

### 1.2) Herstellung des Metathesekatalysators der Formel 1a.

In einem Schlenkrohr wurden 424 mg (0,5 mmol) Grubbskatalysator, 2. Generation, 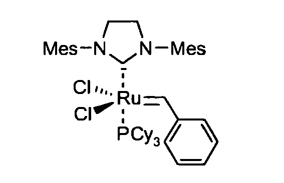 und 59 mg CuCl (0,6 mmol) vorgelegt, Unter Argon wurde die Lösung von 134 mg (0,6 mmol) 2-(2-Propenyl-phenyloxy)propionsäuremethylester , gelöst in 10 ml CH₂Cl₂, zugegeben. Die Mischung wurde 1 Stunde bei 40° C gerührt, danach i.V eingeengt, der Rückstand mit 20 ml Ethylacetat aufgenommen u. die erhaltene trübe Lösung filtriert. Das nach Abdampfen des Lösungsmittels erhaltene Rohprodukt wurde durch zweimalige Chromatographie gereinigt, (Kieselgel Merck Typ 9385, 1.Eluent AcOEt / Cyclohexan 3:7, 2.Eluent AcOEt / Cyclohexan 1:1). Es wurden 193 mg (58 % d. Th.) Reinprodukt erhalten.
HRMS(El): C₃₂H₃₈N₂O₃Cl₂Ru
Berechnet: [M⁺]670.13030, gef.: 670.13467

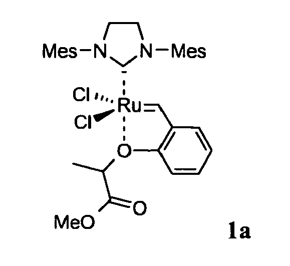

### BEISPIEL 2

Verwendung des erfindungsgemäß hergestellten Katalysators der Formel **1a** und Vergleich seiner Aktivität mit dem bekannten Katalysator der Formel A (siehe oben).
2.1) Ringschlussmetathese (RCM) unter Verwendung von **1a:**
   Zu einer Lösung von 100 mg (0,4 mmol) Allyl-methallyl-malonsäurediethylester (Substrat) in 20 ml Dichlormethan wurde eine Lösung von 2,7mg (0,004 mmol) des nach Beispiel 1 erhaltenen Katalysators **1a** in 1 ml Dichlormethan bei 25°C hinzugefügt. Die Reaktionsmischung wurde 2 Stunden bei dieser Temperatur gehalten. Nach dieser Zeit wurde eine Probe entnommen, der Katalysator durch Zugabe von Ethylvinylether zerstört und die Probe gaschromatographisch analysiert, (Vergleich mit Substrat und in bekannter Weise hergestelltem Ringschlussprodukt). Der Umsatz des Substrates zum Metatheseprodukt (1,1-Bis-ethoxycarbonyl-3-methyl-cyclopent-3-en) betrug 89%.
2.2) RCM unter Verwendung des bekannten Katalysators der Formel **A:**
   In gleicher Weise wie im Beispiel 2.1 beschrieben, jedoch unter Verwendung von 2,5 Mol% des bekannten Katalysators A, wurde das gleiche Substrat umgesetzt. Die gaschromatographische Untersuchung ergab, dass der Umsatz zum Ringschlussprodukt 18% betrug.

### BEISPIEL 3

Verwendung des erfindungsgemäß hergestellten Katalysators der Formel **1a** und Vergleich seiner Aktivität mit dem bekannten Katalysator der Formel **D** (siehe oben). 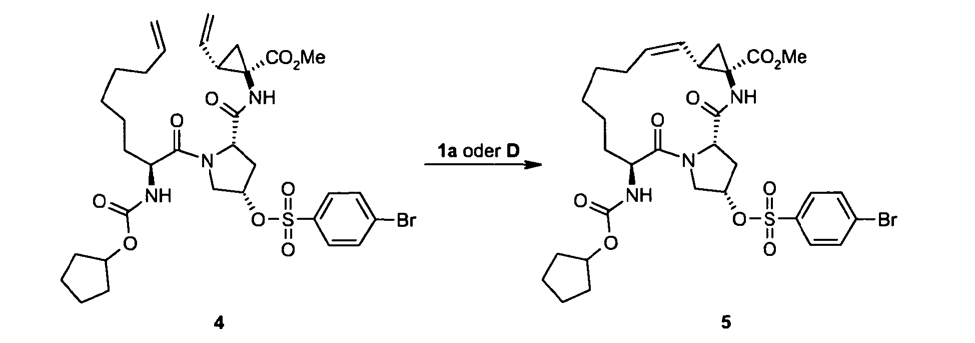 **4** wird in 10 ml entgastem Toluol gelöst und auf 80°C erhitzt, frisch hergestellter Katalysator wird unter Stickstoff zugegeben (1^{st}) und die entstandene Mischung für 60 min bei 80°C gerührt. Die Umsetzung wird per HPLC geprüft und eine weitere Menge des Katalysators zugegeben (2^{nd}). Nach weiteren 60 min wird die Umsetzung noch einmal per HPLC geprüft.

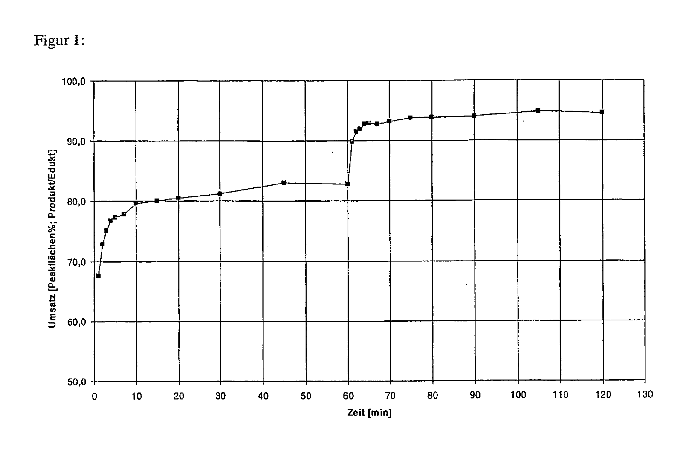

Figur 1 zeigt die Cyclisierungsgeschwindigkeit von **4** (T = 0 min:0,4 mol%; T = 60 min:0,2 mol%). Tabelle 1 zeigt die Ergebnisse einer HPLC Analyse wobei das Ergebnis mit dem bekannten Katalysator der Formel **D** verglichen wird. (Angew. Chem. Int. Ed. 2002, 41, 4038).

**Table 1**

| | | | | Umsetzung [%Peak at 200nm] | |
|---|---|---|---|---|---|
| Katalysator | 1^{st} [mol%] | **4** [mol] | Toluol [ml] | **5** [60 min] | **4** [60 min] |
| | 2^{nd} [mol%] | | | **5** [120 min] | **4** [120 min] |
| **1a** | 0,4 | 0,0075 | 554 | 82,8 | |
| | 0,2 | | | 94,6 | |
| **D** | 0,4 | 0,015 | 1008 | 77,4 | 22,6 |
| | 0,2 | | | 92,8 | 7,2 |

## Patentansprüche

1. Verbindungen der allgemeinen Formel **1**, worin 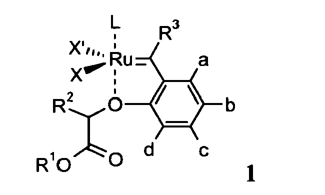
X und X' anionische Liganden;
L neutraler Ligand;
a, b, c, d unabhängig voneinander H, -NO₂, C₁₋₁₂-Alkyl, C₁₋₁₂-Alkoxy oder Phenyl, wobei Phenyl gegebenenfalls mit einem Rest ausgewählt aus der Gruppe C₁₋₆-Alkyl und C₁₋₆-Alkoxy substituiert sein kann;
R¹ C₁₋₁₂-Alkyl, C₅₋₆-Cycloalkyl, C₇₋₁₈-Aralkyl, Aryl;
R² H, C₁₋₁₂-Alkyl, C₅₋₆-Cycloalkyl, C₇₋₁₈-Aralkyl, Aryl;
R³ H, C₁₋₂₀-Alkyl, C₂₋₂₀-Alkenyl, C₂₋₂₀-Alkinyl, Aryl;
bedeuten.

2. Verbindungen der allgemeinen Formel **1** nach Anspruch 1, worin
a, b, c H; und
d Phenyl mit einem Rest ausgewählt aus der Gruppe C₁₋₆-Alkyl und C₁₋₆-Alkoxy substituiert;
bedeuten.

3. Verbindungen der allgemeinen Formel 1 nach einem der vorangegangenen Ansprüche 1 oder 2, worin
a, c, d H; und
b -NO₂;
bedeuten.

4. Verbindungen der allgemeinen Formel **1** nach einem der vorangegangenen Ansprüche 1-3, worin
L für einen Liganden der Formel P(R⁴)₃ steht, worin R⁴ C₁₋₆-Alkyl, Cycloalkyl oder Aryl
bedeutet.

5. Verbindungen der allgemeinen Formel **1** nach einem der vorangegangenen Ansprüche 1-3, worin L ein Liganden der Formel L¹, L², L³ oder L⁴ bedeutet, 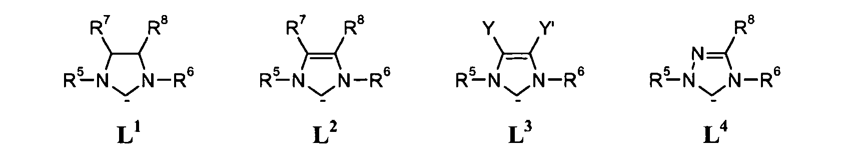 worin
R⁵ und R⁶ unabhängig voneinander H, C₁₋₆-Alkyl oder Aryl;
R⁷ und R⁸ unabhängig voneinander H, C₁₋₆-Alkyl, C₂₋₆-Alkenyl oder Aryl; oder
R⁷ und R⁸ zusammen eine 3- oder 4-gliedrige Alkylenbrücke bilden; und
Y und Y' Halogen;
bedeuten.

6. Verbindungen der allgemeinen Formel **1** nach Anspruch 5, worin
X und X' Cl;
L L¹;
a, b, c, d H;
R¹ Methyl;
R² Methyl;
R³ H;
R⁵ und R⁶ Mesityl
R⁷und R⁸ H;
bedeutet.

7. Verfahren zur Durchführung von Metathesereaktionen, in dem zwei Verbindungen zur Reaktion gebracht werden, die je eine olefinische Doppelbindung enthalten oder eine der Verbindungen mindestens zwei olefinische Doppelbindungen enthält und in dem als Katalysator eine der Verbindungen gemäß den Ansprüchen 1 bis 6 verwendet wird.

8. Verfahren zur Durchführung einer Ringschlussmetathese (RCM) oder einer Kreuzmetathese (CM) an dem eine Verbindung, die zwei olefinische Doppelbindungen enthält, als Substrat und eine der Verbindungen gemäß den Ansprüchen 1 bis 6 als Katalysator beteiligt ist.

## Claims

1. Compounds of general formula **1**, wherein 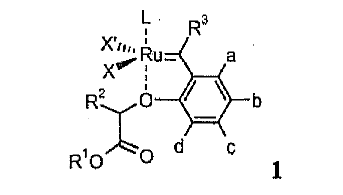
X and X' denote anionic ligands;
L denotes a neutral ligand;
a, b, c, d independently of one another denote H, -NO₂, C₁₋₁₂-alkyl, C₁₋₁₂-alkoxy or phenyl, while phenyl may optionally be substituted by a group selected from among C₁₋₆-alkyl and C₁₋₆-alkoxy;
R¹ denotes C₁₋₁₂-alkyl, C₅₋₆-cycloalkyl, C₇₋₁₈-aralkyl, aryl;
R² denotes H, C₁₋₁₂-alkyl, C₅₋₆-cycloalkyl, C₇₋₁₈-aralkyl, aryl;
R³ denotes H, C₁₋₂₀-alkyl, C₂₋₂₀-alkenyl, C₂₋₂₀-alkynyl, aryl.

2. Compounds of general formula 1 according to claim 1, wherein
a, b, c denote H; and
d denotes phenyl substituted by a group selected from among C₁₋₆-alkyl and C₁₋₆-alkoxy.

3. Compounds of general formula **1** according to one of the preceding claims 1 or 2, wherein
a, c, d denote H; and
b denotes -NO₂.

4. Compounds of general formula **1** according to one of the preceding claims 1-3, wherein
L denotes a ligand of formula P(R⁴)₃ wherein R⁴ denotes C₁₋₆-alkyl, cycloalkyl or aryl.

5. Compounds of general formula **1** according to one of the preceding claims 1-3, wherein L denotes a ligand of formula L¹, L², L³ or L⁴, 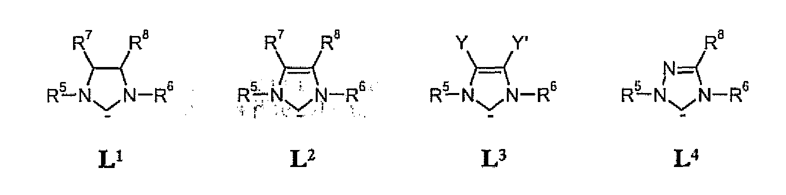 wherein
R⁵ and R⁶ independently of one another denote H, C₁₋₆-alkyl or aryl;
R⁷ and R⁸ independently of one another denote H, C₁₋₆-alkyl, C₂₋₆-alkenyl or aryl; or
R⁷ and R⁸ together form a 3- or 4-membered alkylene bridge; and
Y and Y' denote halogen.

6. Compounds of general formula **1** according to claim 5, wherein
X and X' denote Cl,
L denotes L¹;
a, b, c, d denote H;
R¹ denotes methyl;
R² denotes methyl;
R3 denotes H;
R⁵ and R⁶ denote mesityl
R⁷ and R⁸ denote H.

7. Process for carrying out metathesis reactions, in which two compounds are reacted, each of which contains an olefinic double bond or wherein one of the compounds contains at least two olefinic double bonds and wherein one of the compounds according to claims 1 to 6 is used as catalyst.

8. Process for carrying out a ring-closing metathesis (RCM) or a cross metathesis (CM) in which a compound containing two olefinic double bonds participates as the substrate and one of the compounds according to claims 1 to 6 participates as the catalyst.

## Revendications

1. Composés de formule générale **1** 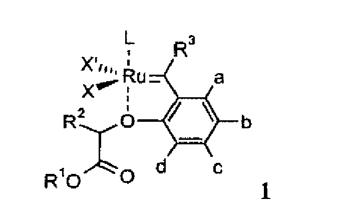 où
X et X' représentent des ligands anioniques ;
L représente un ligand neutre ;
a, b, c, d représentent indépendamment les uns des autres H, -NO₂, C₁₋₁₂-alkyle, C₁₋₁₂-alcoxy ou phényle, où phényle peut éventuellement être substitué avec un groupement choisi dans le groupe C₁₋₆-alkyle et C₁₋₆-alcoxy ;
R¹ représente C₁₋₁₂-alkyle, C₅₋₆-cycloalkyle, C₇₋₁₈-aralkyle, aryle ;
R² représente H, C₁₋₁₂-alkyle, C₅₋₆-cycloalkyle, C₇₋₁₈-aralkyle, aryle ;
R³ représente H, C₁₋₂₀-alkyle, C₂₋₂₀-alcényle, C₂₋₂₀-alcynyle, aryle.

2. Composés de formule générale **1** selon la revendication 1, où a, b, c représentent H ; et
d représente phényle substitué avec un groupement choisi dans le groupe C₁₋₆-alkyle et C₁₋₆-alcoxy.

3. Composés de formule générale **1** selon l'une des revendications 1 ou 2 précédentes, où
a, c, d représentent H ; et
b représente -NO₂.

4. Composés de formule générale **1** selon l'une des revendications 1-3 précédentes, où
L représente un ligand de formule P(R⁴)₃ où R⁴ représente C₁₋₆-alkyle, cycloalkyle ou aryle.

5. Composés de formule générale **1** selon l'une des revendications 1-3 précédentes, où L représente un ligand de formule L¹, L², L³ ou L⁴, 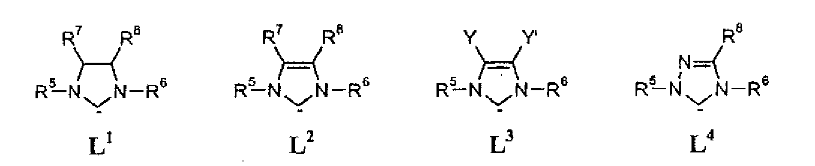 où
R⁵ et R⁶ représentent indépendamment l'un de l'autre H, C₁₋₆-alkyle ou aryle;
R⁷ et R⁸ représentent indépendamment l'un de l'autre H, C₁₋₆-alkyle, C₂₋₆-alcényle ou aryle; ou
R⁷ et R⁸ forment ensemble un pont alkylène à 3 ou 4 chaînons ; et
Y et Y' représentent un halogène.

6. Composés de formule générale **1** selon la revendication 5 où
X et X' représentent Cl ;
L représente L¹ ;
a, b, c, d représentent H ;
R¹ représente méthyle ;
R² représente méthyle ;
R³ représente H ;
R⁵ et R⁶ représentent mésityle ;
R⁷ et R⁸ représentent H.

7. Procédé pour conduire des réactions de métathèse où deux composés sont mis à réagir, qui contiennent chacun une double liaison oléfinique ou l'un des composés contient au moins deux doubles liaisons oléfiniques et où l'un des composés selon les revendications 1 à 6 est utilisé comme catalyseur.

8. Procédé pour conduire une métathèse avec cyclisation (RCM) ou une métathèse croisée (CM) auquel participent un composé qui contient deux doubles liaisons oléfiniques comme substrat et l'un des composés selon les revendications 1 à 6 comme catalyseur.
